# EUROPEAN PATENT SPECIFICATION

(11) **EP 4 243 773 B1**
(45) Date of publication and mention of the grant of the patent: **09.09.2026**
(21) Application number: 21819578.2
(22) Date of filing: 12.11.2021
(51) Int. Cl.: A61K 9/10, A61K 47/24, A61K 47/44

(54) **SOLID DISPERSIONS OF CANNABIDIOL**
FESTE DISPERSIONEN VON CANNABIDIOL
DISPERSIONS SOLIDES DE CANNABIDIOL

(30) Priority: 16.11.2020 IT 202000027408
(43) Date of publication of application: 20.09.2023
(73) Proprietor: Indena S.p.A., 20139 Milano (IT)
(72) Inventor: ALLEGRINI, Pietro, 20139 Milano (MI) (IT); RONCHI, Massimo, 20139 Milano (MI) (IT); RIVA, Antonella, 20139 Milano (MI) (IT)
(74) Representative: Bianchetti & Minoja with Trevisan & Cuonzo IPS SRL
(86) International application number: PCT/IB2021/060499
(87) International publication number: WO 2022/101841

(56) References cited:
- WO-A1-2018/152334
- WO-A1-2020/014776
- WO-A1-2020/016653
- CN-A- 111 991 444
- US-A1- 2018 250 262

## Description

### Field of invention

The present invention relates to a composition comprising cannabidiol and at least two excipients, and to the preparation process of the composition and to pharmaceutical or nutraceutical formulations containing it.

### Background to the invention

The term "cannabinoid" defines a family of natural, semi-synthetic or synthetic compounds which share the ability to bind to certain receptors (called "cannabinoid receptors") coupled to protein G, the stimulation whereof inhibits the enzyme adenylate-cyclase and therefore the production of cyclic AMP; said family of compounds derives its name from *Cannabis sativa,* a botanical species widely used in traditional medicine in various countries.

Among the hundreds of natural cannabinoids, also known as phytocannabinoids, which are present in plants belonging to the various species of *Cannabis spp.,* the two priority compounds are Δ⁹-tetrahydrocannabinol 1, tetrahydro-6,6,9-trimethyl-3-pentyl-6H-dibenzo[b,d]pyran-1-ol, hereinafter called "THC 1", and cannabidiol 2, 2-[(1R,6R)-3-methyl-6-(prop-1-en-2-yl)-cyclohex-2-enyl]-5-pentylbenzen-1,3-diol, hereinafter called "CBD 2".

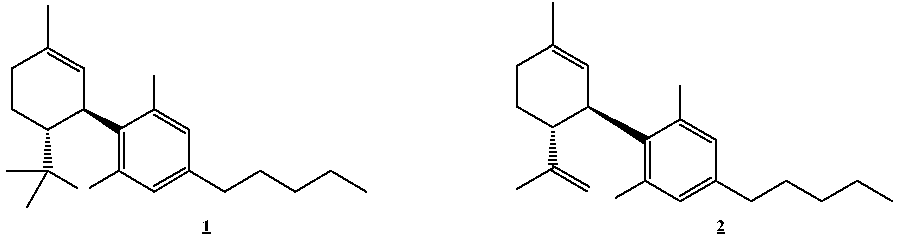

CBD 2, which is approved for the treatment of convulsions associated with Lennox-Gastaut syndrome and Dravet syndrome, has exhibited such promising therapeutic effects that it has been tested in clinical trials for use as an anti-inflammatory, analgesic, antiemetic, neuroprotective, anxiolytic, etc..

However, despite its promising therapeutic effects, the low bioavailability of cannabidiol 2, due to its lipophilic nature, represents the main problem in the development of oral formulations; in fact, cannabidiol 2 is absorbed with difficulty in the aqueous medium of the gastrointestinal tract because of its low, uneven solubilisation. Moreover, the absorption of cannabidiol 2 is further reduced, due to the first-pass metabolism (or first-pass effect), to an oral bioavailability value of about 6% (Izgelov D. et al, Eur. J. Pharm. Biopharm. 2020 154 108).

Various formulation approaches have been used to improve the solubility and bioavailability of cannabidiol 2, including the preparation of solutions containing CBD 2 combined with lipid excipients (surfactants or triglycerides), as described in international patent application WO 2018/152334, or solutions containing CBD 2 encapsulated in phospholipid vesicles, as described in patent application US 2020/0078427; in further formulation examples, CBD 2 is combined with phospholipids in solid or semisolid formulations, as described in international patent application WO 2017/098502, prepared without the use of further fats or solvents.

Published international patent WO-2020014776 discloses an oral dispersible film strip comprises 0,1 to 40 wt% of oil used, between 10: 1 to 1 : 10 weight ratio of cannabinoid (purified CBD) : oil, 30-80 wt% film-forming agent like HPMC or pullulan, 0,5-20 wt% of surfactants like lecithin, glycolipids, fatty alcohols, fatty acids, esters of fatty alcohols and fatty acids, sorbitan esters, polyols such as polysorbates, sorbitans, or long-chain aliphatic acids.

The Chinese patent CN-111991444 discloses a solubilized hemp oil solid dispersion comprising 1 part of hemp oil(cannabis oil includes cannabinoids like cannabidiol (CBD); 3-10 parts by weight selected from the group consisting of a hydrophilic-lipophilic balance (HLB value) between 13-23. The solubilized carrier composition comprising a) 3-10 parts surfactant like polyethylene glycol 1000 vitamin E succinate (TPGS), b) less than 5 parts high molecular polymer, like Hydroxypropyl cellulose, and c) optionally comprising 0.01-0.05 parts by weight of antioxidant. The preparation method, the organic solvent must be able to dissolve the surfactant, polymer auxiliary material, and antioxidant in the solubilizing carrier composition.

Supramolecular complexes of CBD 2 and phospholipids (such as micelles, vesicles, etc.) form in said formulations due to their high lipid content; said complexes, by interaction with chylomicrons, are conveyed in the lymphatic system directly from the intestine, thereby avoiding metabolisation in the liver.

Although chylomicron-mediated absorption enables the first-pass metabolism to be avoided, partly increasing the bioavailability of CBD 2, lymphatic absorption presents the following main drawbacks: long absorption times and limited tissue distribution of CBD 2, mainly in the muscle and adipose tissue.

In view of these factors, there is clearly a need to develop new formulations containing CBD 2 wherein not only the bioavailability, but also the absorption rate and tissue distribution, are increased.

### Description of the invention

The present invention relates to solid dispersions comprising cannabidiol 2 and at least two or more excipients, the associated preparation process, and pharmaceutical or nutraceutical formulations containing them.

In a first aspect thereof, the present invention relates to a composition in the form of a solid dispersion, typically a solid powder dispersion, comprising CBD 2 and at least two excipients, wherein at least one excipient ("excipient L") is a lipid excipient and wherein at least one excipient ("excipient I") is a hydrophilic excipient, and wherein excipient L is present in an amount equal to or lower than 20% of the total weight of the composition.

For the avoidance of doubt, the term "excipient L" identifies a lipid excipient or a mixture of lipid excipients; if excipient L in the composition is a mixture of lipid excipients, the amount of each excipient can be the same as or different from the others, and in any event their total amount is equal to or lower than 20% of the total weight of the composition; the term "excipient I" identifies a hydrophilic excipient or a mixture of hydrophilic excipients; if excipient I in the composition is a mixture of hydrophilic excipients, the amount of each excipient can be the same as or different from the others, and in any event their total amount is equal to or lower than 40% of the total weight of the composition. Moreover, again for the avoidance of doubt, excipient L is present in amounts other than 0 (zero), preferably ranging from 1%-20%, 5%-20% or 10-20% of the total weight of the composition; excipient I is present in amounts other than 100%, preferably ranging from 40%-98%, 40-90%, 40-80%, 40-70% or 40%-60% of the total weight of the composition.

In the present invention, the term "solid dispersion" defines a system of two or more components, wherein a first component (in the particular case of the present invention, cannabidiol 2) is distributed homogeneously in a second component or in a mixture of components (in this particular case, in excipients L and I); in the resulting, physically uniform system, there is no formation of supramolecular complexes such as micelles, liposomes or microemulsions.

In addition to excipients L and I and cannabidiol 2, the solid dispersion according to the invention can also comprise further excipients, typically surfactants and/or lubricants and/or glidants; as a whole, the sum of the individual amounts of cannabidiol 2 and the further excipients is equal to or lower than 40% of the total weight of the composition.

In a preferred aspect thereof, the composition according to the invention comprises CBD 2, excipient L, excipient I and a surfactant.

In a second aspect thereof, the invention relates to a process for preparation of the solid powder dispersion of CBD 2, comprising the use of an organic solvent.

In particular, the process involves initial mixing, in the presence of an organic solvent, of CBD 2, excipient L and excipient I, whereafter the solid dispersion is obtained by removing the solvent.

In a third preferred aspect thereof, the invention relates to oral pharmaceutical or nutraceutical formulations containing the compositions in the form of a solid powder dispersion of CBD 2.

Excipient L is a sunflower or soy lecithin containing phosphatidylcholine in the amount of not less than 25%; preferably, excipient L is a sunflower or soy lecithin containing phosphatidylcholine in the amount of not less than 90%. For example, excipient L is a soy lecithin containing phosphatidylcholine in the amount of not less than 90%, commercially available as Epikuron^{™} 200.

Typically, excipient I is selected from cellulose, microcrystalline cellulose, methylcellulose, ethylcellulose, hydroxymethylcellulose, hydroxyethylcellulose, hydroxypropylcellulose, hydroxypropyl methylcellulose, carboxymethylcellulose, carboxymethylethylcellulose, starch, pectins, maltodextrins, cyclodextrins, sucrose, fructose, sorbitol, mannitol, maltitol, xylitol, dextrose, trehalose, inulin, carrageenan, alginate, gum arabic and guar gum; in a most preferred aspect thereof, excipient I is hydroxypropyl methylcellulose or microcrystalline cellulose or a combination thereof.

In a preferred embodiment, excipient I is present in amounts equal to or greater than 40% of the total weight of the composition.

Typically, the surfactant is selected from cetyl alcohols, glycerol monostearates, polyethylene glycol stearates, polysorbates, polyoxyethylene/polyoxypropylene copolymer, d-alpha tocopheryl polyethylene glycol 1000 succinate, polysorbates, macrogol^{™}, 15-hydroxystearate, PEG 40 hydrogenated castor oil, polyglyceride esters of fatty acids and mixtures thereof; preferably, the surfactant is a polyethylene glycol stearate.

In a preferred embodiment, the solid dispersion according to the invention comprises CBD 2, a sunflower or soy lecithin containing phosphatidylcholine in the amount of not less than 25%, and at least two celluloses or cellulose derivatives and a surfactant.

In a further preferred embodiment, the solid dispersion according to the invention comprises CBD 2, a sunflower or soy lecithin containing phosphatidylcholine in the amount of not less than 25%, at least two celluloses or cellulose derivatives, a surfactant and a lubricant and/or glidant.

In a most preferred embodiment, the solid dispersion according to the invention comprises CBD 2, a sunflower or soy lecithin containing phosphatidylcholine in the amount of not less than 90%, at least two celluloses or cellulose derivatives, a surfactant and a lubricant and/or glidant.

As regards the second aspect of the invention, the process preferably comprises the following steps:
a) excipient L is suspended in an organic solvent to give a suspension (suspension A);
b) suspension A is heated and maintained under stirring until excipient L dissolves, to give a solution (solution B);
c) solution B is heated and maintained under stirring, and CBD 2 is added to give a solution (solution C);
d) solution C is heated and maintained under stirring, and excipient I is added to give a suspension (suspension D);
e) the solvent of suspension D is removed, to give a solid (solid E);
f) a lubricant and/or glidant is optionally added to solid E, to give a solid (solid F);
g) optional grinding of solid E or F to give a powder.

Depending on the pharmaceutical or nutraceutical formulation wherein the solid dispersion is to be used, a process comprising steps a)-e) only, or also the optional steps f)-g), can be used; a process comprising steps a)-e) gives rise to a solid dispersion in granulate form, the granules whereof have variable, heterogeneous sizes; conversely, a process comprising steps a)-g) gives rise to a solid powder dispersion with a homogeneous particle size, usually ranging between 10 and 500 µm.

In particular, the ratio between CBD 2 and excipient L used in step a) is greater than or equal to 0.5 and less than 10; in particular, the ratio between CBD 2 and excipient L is greater than 2 and less than 5; preferably, the ratio between cannabidiol 2 and excipient L is 2.5.

When a mixture of excipients I is used in step d), in particular if two excipients I of different amounts are used, the excipient present in the larger amount constitutes at least 40% of the total weight of the composition, whereas the one present in the smaller amount is present in an amount not exceeding 20%, and preferably in an amount not exceeding 10%.

Typically, the ratio between cannabidiol 2 and the one or more excipients I falls into the range between 0.1 and 10; in particular, in the case of two or more excipients I, the ratio between cannabidiol 2 and the excipient I present in the larger amount falls into the range between 0.1 and 2, while the ratio between CBD 2 and the one or more excipients I present in the smaller amount falls into the range between 2 and 8; preferably, the preferred ratios between CBD 2 and excipients I range between 0.3 and 0.7 and between 3.5 and 6 for the excipient I present in the larger and smaller amount respectively.

In step a), the solvent used is selected from methanol, ethanol, propanol, isopropanol, acetone, ethyl acetate, tetrahydrofuran and dioxane; preferably, the solvent used is selected from ethanol and ethyl acetate.

In step a), in addition to excipient L, a surfactant can also be optionally suspended in the organic solvent; preferably, the ratio between CBD 2 and the optional surfactant ranges between 0.5 and 10; and more preferably, between 1.5 and 5.

Typically, in step b), suspension A is maintained under stirring and heating until partial or complete dissolution of the lipid excipient; preferably until it has completely dissolved.

In steps b)-d) the temperature is maintained in the range between 25 and 130° C; in particular the temperature is maintained in the range between 50 and 90°C; and even more in particular the temperature is maintained between 60 and 70°C.

In step d), a technique selected from distillation, low-pressure distillation and spray drying is used to remove the solvent; preferably, low-pressure distillation.

In step f), the lubricant and/or glidant used is selected from stearic acid, magnesium stearate, silica and talc; preferably silica.

In particular, the lubricant and/or glidant is present in an amount not exceeding 5% of the total weight of the composition.

In step g), solid F is ground to a particle size ranging between 10 and 500 µm.

In the process according to the invention, none of steps a)-g) involve the use of water, for example for solubilisations, dilutions or suspensions.

Moreover, in the present invention, none of steps a)-g) involves the addition of an oily carrier such as a synthetic, semisynthetic or natural oil.

The solubility of the solid powder dispersion of CBD 2, prepared as described in Example 1, was evaluated in simulated biological fluids. In particular, comparative solubility studies have been conducted between the solid powder dispersion of cannabidiol 2, a mechanical mixture with a composition similar to the solid powder dispersion of CBD 2, and non-formulated CBD 2, in a fasted-state simulated intestinal fluid at pH= 6.5 (hereinafter called FaSSIF) and in a fed-state simulated intestinal fluid at pH= 5 (hereinafter called FeSSIF). For the purposes of the present description, the term "mechanical mixture" means a combination of one or more solids amalgamated by mixing techniques not involving the use of any solvent.

As will be seen from the results set out in Table 2 to Example 3, in FaSSIF, an increase in the solubility of CBD 2 is observed in the solid powder dispersion, amounting to 161% compared with the non-formulated CBD 2 and 124% compared with the cannabidiol 2 of the mechanical mixture. In FaSSIF, an increase in the solubility of CBD 2 is observed in the solid powder dispersion, amounting to 64% compared with the non-formulated CBD 2 and 61% compared with the cannabidiol 2 of the mechanical mixture.

The results of the solubility studies demonstrate that the solubility of CBD 2 considerably increased, not only compared with the non-formulated CBD 2, but also compared with the CBD 2 contained in the mechanical mixture; this surprising result demonstrates that mere mixing of CBD 2 with excipients is insufficient to increase their solubility significantly, and that it is critical to obtain a homogeneous distribution of CBD 2, as in the case of the solid dispersion according to the invention.

Without being bound by any theoretical constraints, the increased solubility of the cannabidiol 2 present in the solid dispersion is attributable to the use of an organic solvent in the preparation process; in fact, the solubilisation of the various ingredients of the composition conducted in steps b)-d) gives rise to a homogeneous solid dispersion wherein cannabidiol 2 is uniformly present in the dispersing mixture.

Moreover, the fact that excipient L is present in an amount equal to or lower than 20% of the total weight of the composition increases the solubility of cannabidiol 2, which has a lipophilic nature, due to excipient-cannabidiol 2 interaction, but without leading to the formation of supramolecular complexes such as micelles, microemulsions or liposomes. The presence of said complexes is disadvantageous because it adversely affects the absorption rate, due to the need to "release" the active ingredients they contain. In view of these factors, comparative experiments were conducted to establish the possibility of further reducing the amount of excipient L, compared with the solid dispersion of Example 1, to reduce the probability of formation of said supramolecular complexes.

As shown in Table 1, both the dispersion of Example 4 and the dispersion of Example 5 contain a smaller amount of excipient L than the dispersion of Example 1; moreover, the dispersion of Example 5 contains a further surfactant.

**Table 1**

| | Example 1 | Example 4 | Example 5 |
|---|---|---|---|
| CBD 2 | 10% | 25% | 25% |
| Excipient L | 20% | 15% | 10% |
| Surfactant | --- | --- | 8% |
| Excipient I | 63% | 53% | 46% |
| Excipient I | 5% | 5% | 6% |
| Glidant | 2% | 2% | 5% |

The solubility of the solid powder dispersions of cannabidiol 2, prepared as described in Examples 4 and 5, was evaluated in FaSSIF; as will be seen from the results set out in Table 3 to Example 6, the solubility of cannabidiol 2 in the solid dispersions of Examples 4 and 5 is increased, compared with non-formulated cannabidiol 2, by 37% and 452% respectively. It is postulated that the difference between the solubility of the cannabidiol 2 of the solid dispersion according to Example 4, and that of the cannabidiol 2 of the solid dispersion according to Example 5, is attributable to the use of not less than 90% of a lecithin containing phosphatidylcholine (Epikuron^{™} 200), and to the presence of the surfactant.

The solid powder dispersion of cannabidiol 2 prepared according to Example 5 was used in an *in vivo* pharmacokinetic study to evaluate its absorption rate and mechanism; the study was conducted on the following formulations:
- Formulation 1: Cannabidiol 2 in a solution of sesame oil and anhydrous ethanol;
- Formulation 2: Cannabidiol 2 in an aqueous suspension containing sodium carboxymethylcellulose (1%) and polysorbate 80 (0.25%)
- Formulation 3: Solid dispersion of cannabidiol 2, according to Example 5, in aqueous suspension containing sodium carboxymethylcellulose (1%)

The concentration of THC 1, CBD 2 and 7-hydroxy-cannabidiol 3, 2-[(1*R*,6*R*)-3-(hydroxymethyl)-6-(1-methylethenyl)-2-cyclohexen-1-yl]-5-pentyl- 1,3-benzenediol, hereinafter called "7-OH-CBD 3", was analysed in the plasma of male Sprague Dawley rats, one day before and 0.25, 0.50, 1, 2, 3, 6 and 24 hours after administration in a single dose, equivalent to 50 mg/kg of cannabidiol 2, of Formulations 1-3.

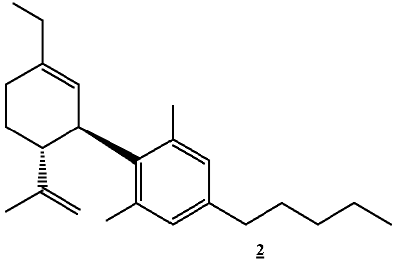

As will be seen from the results of the pharmacokinetic study shown in Table 4 of Example 8, as regards the plasma concentrations of CBD 2, the peak maximum concentration (Cₘₐₓ) is surprisingly reached about 3 hours after administration of Formulations 2 and 3, whereas for Formulation 1, said peak is reached 6 hours after treatment.

It will also be seen that the administration of Formulation 1 gives rise to a plasma CBD 2 concentration greater than that reached after administration of Formulation 3, which in turn is greater than that obtained by administering Formulation 2.

Without espousing any particular theories, the differences in maximum concentrations and the times taken to reach them which were measured in the *in vivo* pharmacokinetic study for the three different formulations are attributable to their different contents of excipient L: in fact, the presence of an excipient L not only affects the solubility of CBD 2 by increasing it, and consequently increasing its bioavailability (as demonstrated by the results of the solubility studies set out above) but, depending on the amount, can also affect the CBD 2 absorption mechanism. For example, the presence of sesame oil in Formulation 1 gives rise to an overall delay in CBD 2 absorption, causing a reduction in stomach voiding time at gastric level and the onset of a lipid digestion process at intestinal level, leading to absorption of CBD 2 via chylomicrons. Said absorption mechanism is not only slow, but also involves a smaller distribution volume of CBD 2: while on the one hand, direct access to the lymphatic system from the intestine enables CBD 2 to avoid being metabolised in the liver, on the other hand the large size of the chylomicrons limits their capillary absorption, leading to an uneven tissue distribution of CBD 2, favouring tissues such as adipose and muscle tissue over others, such as nerve tissue.

Formulation 3, containing the solid powder dispersion of CBD 2 according to the present invention, exhibits a different absorption mechanism from that of Formulation 1, via chylomicrons, and that of Formulation 2; in fact, Table 6 of Example 8 shows two absorption peaks for the CBD 2 of Formulation 3, measured 1 hour and 3 hours after administration, corresponding to two intestinal absorption windows, the first in the small intestine and the second in the distal part or in the caecum and/or the proximal colon. Although part of the CBD 2 absorbed by said mechanism is metabolised by the liver, as confirmed by the comparison between the 7-OH-CBD 3/CBD 2 concentration ratios set out in Table 6 of Example 8, the remainder is absorbed more rapidly and distributed in higher volumes and more evenly between the various tissues than the CBD 2 of Formulations 1 and 2.

The rapid absorption of Formulation 3 is particularly advantageous; in view of the short absorption times, the solid powder dispersion of cannabidiol 2 could be used, for example, in analgesic, anti-inflammatory or anxiolytic pharmaceutical preparations or, more generally, in the treatment of conditions requiring a rapid therapeutic response. Moreover, as the tissue distribution of the CBD 2 in Formulation 3, for example in nerve tissue, is greater than that of formulations containing oils, such as Formulation 1 and the commercial product Epidiolex^{™}, it can be postulated that CBD 2 exhibits a different pharmacological response from the known response.

With special reference to the third aspect of the invention, examples of oral formulations include, but are not limited to, tablets, chewable tablets, capsules, soft gelatin capsules, hard gelatin capsules, bars, candies, cereals, cereal coatings and combinations thereof. The formulations can be prepared by known methods and with known ingredients, for example as described in Remington: "The Science and Practice of Pharmacy", 22nd edition, Pharmaceutical Press, 2013.

Examples of said ingredients include, but are not limited to, disintegrants, lubricants, binders, coating agents, colourings, absorption promoters, solubilising agents, stabilisers, flavourings, sweeteners, antiseptics, preservatives, antioxidants, etc..

### Experimental section

### Materials and methods

The materials used in the preparation of the solid dispersions according to the invention and cited in the following experimental part in Examples 1-8 are all commercially available.

The results of the pharmacokinetic study reported in Example 8 were obtained by LC-MS analysis of biological samples using an Exion LC 100 system connected to an API 4000 triple-quadrupole mass spectrometer equipped with a TurboV electrospray interface.

The chromatographic separations were conducted with an Agilent Zorbax^{™} SB-C18 column, with Agilent Zorbax^{™} pre-column, maintained at 40°C; an eluent mixture according to the following gradient was used in the chromatographic analysis:

| Minutes | Phase A (ammonium acetate) | Phase B (acetonitrile) |
|---|---|---|
| 0-5 min. | From 70% to 10% | From 30% to 90% |
| 5-10 min. | 10% | 90% |
| 10-13 min. | 70% | 30% |

The mass spectrometer was used in negative-ion mode, applying an ionisation potential of -4.5kV.

### Examples

### Example 1 (according to the invention) - Solid powder dispersion of CBD 2 (type 1)

20 g of sunflower lecithin (Emulpur^{™}/SF) was suspended in EtOH (3000 mL); the resulting suspension was heated to 70°C and maintained under stirring for 30 min., to promote dissolution of the lecithin. 10 g of cannabidiol 2, 63 g of microcrystalline cellulose and 5 g of hydroxypropyl methylcellulose were added to the resulting solution; after 30 min. under stirring and heating, the solvent was removed from the suspension by low-pressure distillation. 2 g of silica was added to the resulting solid, weighing 98 g. The resulting solid mixture was ground to a particle size ranging between 10 and 500 µm.

### Example 2 (comparative) - Mechanical mixture of CBD 2

10 g of cannabidiol 2, 20 g of sunflower lecithin (Emulpur^{™}/SF), 63 g of microcrystalline cellulose, 5 g of hydroxypropyl methylcellulose and 2 g of silica were mixed until a homogeneous mixture was obtained.

### Example 3 - Solubility studies in simulated gastric fluids [CBD, Solid powder dispersion of CBD 2, Mechanical mixture of CBD 2]

The solubility studies in different biologically relevant media of the solid dispersion prepared according to Example 1 were conducted to evaluate its behaviour in the intestine under fed and fasted conditions. The solubility studies were conducted in FaSSIF (pH 6.5) and FeSSIF (pH 5).

10-20 mL of FaSSIF and FeSSIF were placed in 40 mL glass vials. An amount of solid dispersion prepared according to Example 1, and an amount of mechanical mixture prepared according to Example 2, in excess of the expected saturated solubility, was added to the vials; the resulting mixtures were maintained under stirring for 2 hours at room temperature. The mixtures were filtered through 0.45 µm PTFE membrane filters. The filtered solutions were analysed with HPLC instrumentation.

Table 2 compares the concentrations of cannabidiol 2, the solid dispersion prepared according to Example 1, and the mechanical mixture prepared according to Example 2 in FaSSIF and in FeSSIF.

**Table 2**

| | **Cannabidiol 2 (mg/mL) concentration** | **Percentage increase compared with CBD 2** | **Percentage increase compared with the mechanical mixture of Example 2** |
|---|---|---|---|
| | **FaSSIF** | | |
| Cannabidiol 2 | 0.067 | --- | --- |
| Mechanical mixture according to Example 2 | 0.078 | 16 | --- |
| Solid dispersion according to Example 1 | 0.175 | 161 | 124 |

| | **FeSSIF** | | |
|---|---|---|---|
| Cannabidiol 2 | 0.278 | | |
| Mechanical mixture according to Example 2 | 0.283 | 2 | |
| Solid dispersion according to Example 1 | 0.457 | 64 | 61 |

### Example 4 (according to the invention) - Solid powder dispersion of CBD 2

15 g of sunflower lecithin was suspended in EtOH (3000 mL); the resulting suspension was heated to 70°C and maintained under stirring for 30 min., until the lecithin was completely dissolved. 25 g of cannabidiol 2, 53 g of microcrystalline cellulose and 5 g of hydroxypropyl methylcellulose were added to the resulting solution; after 30 min. under stirring and heating, the solvent was removed from the solution by low-pressure distillation. 2 g of silica was added to the resulting solid, weighing 98 g. The resulting solid mixture was ground to a particle size ranging between 10 and 500 µm.

### Example 5 (according to the invention) - Solid powder dispersion of CBD 2

10 g of soy phosphatidylcholine (Epikuron^{™} 200) and 8 g of polyethylene glycol stearate (Gelucire^{™} 48/16) were suspended in EtOH (3000 mL); the resulting suspension was heated to 70°C and maintained under stirring for 30 min., until the lecithin and polyethylene glycol stearate had completely dissolved. 25 g of cannabidiol 2, 46 g of microcrystalline cellulose and 6 g of hydroxypropyl methylcellulose were added to the resulting solution; after 30 min. under stirring and heating, the solvent was removed from the solution by low-pressure distillation. 5 g of silica was added to the resulting solid, weighing 95 g. The resulting solid mixture was ground to a particle size ranging between 10 and 500 µm.

### Example 6 - Solubility studies in simulated gastric fluids [CBD, Solid powder dispersion of CBD 2, Solid powder dispersion of CBD 2]

The solubility studies in FaSSIF (pH 6.5) of the solid dispersions prepared according to Examples 4 and 5 were conducted to evaluate their behaviour in the intestine under fasted conditions.

10-20 mL of FaSSIF was placed in 40 mL glass vials. An amount of solid dispersion prepared according to Examples 4 and 5, in excess of the expected saturated solubility, was added to the vials; the resulting mixtures were maintained under stirring for 2 hours at room temperature. The mixtures were filtered through 0.45 µm PTFE membrane filters. The filtered solutions were analysed with HPLC instrumentation.

**Table 3**

| Table 3 compares the concentration of cannabidiol 2, the solid dispersion prepared according to Example 4, and the mechanical mixture prepared according to Example 5. | **Cannabidiol 2 (mg/mL) concentration** | **Percentage increase compared with CBD 2** |
|---|---|---|
| Cannabidiol 2 | 0.067 | --- |
| Solid dispersion according to Example 4 | 0.092 | 37 |
| Solid dispersion according to Example 5 | 0.370 | 452 |

### Example 7 - CBD formulations for pharmacokinetic study

The formulations used in the pharmacokinetic study were prepared immediately before administration by the following methods:
- Formulation 1: Cannabidiol 2 was suspended in a 9:1 mixture of sesame oil and anhydrous ethanol. The suspension was maintained under magnetic stirring at room temperature until the CBD 2 had completely dissolved. Final concentration of CBD 2: 10 mg/mL.

- Formulation 2: Cannabidiol 2 was suspended in an aqueous solution of sodium carboxymethylcellulose (1%) and polysorbate 80 (0.25%). Final concentration of CBD 2: 10 mg/mL.
- Formulation 3: A solid powder dispersion of cannabidiol 2 according to Example 5 was suspended in an aqueous solution of sodium carboxymethylcellulose (1%). Final concentration of CBD 2: 38.8 mg/mL.

### Example 8 - Results of pharmacokinetic study

18 male Sprague Dawley rats (aged 6 weeks, body weight between 189 and 229 g) were housed in cages (two animals per cage) with 12-hour light/dark cycles at a temperature of 21.5°C ± 1.5°C and relative humidity of 55% ± 15%. A programme included in the Pristima^{®} package was used to randomise the animals to the treatment groups. The animals were fasted from the previous night until six hours after the treatment. Formulations 1-3, prepared immediately before the treatment, were administered in a single dose using a gastric probe.

After anaesthesia (sevoflurane), blood samples were taken from the retro-orbital plexus before and 0.25, 0.5, 1, 2, 3, 6, and 24 hours after the dose.

The blood samples, collected in heparinised tubes, were immediately placed on ice and kept cold until centrifugation (3 minutes, 10000 rpm, +4°C). At least 150 µL of plasma was taken, divided into two aliquots and stored in the freezer at -80°C until analysis.

**Table 4**

| ***12 h*** | **Tₘₐₓ (h)** | **Cₘₐₓ (ng/mL)** | **AUC _{0-inf}_obs (ng/mL*h)** |
|---|---|---|---|
| **Formulation 1** | 6 (±0) | 1287.97 (±512.24) | 6232.24 (±2355.43)** |
| **Formulation 2** | 3 (±1.55) | 365.07 (±91.08) | 2090.34 (±629.65)** |
| **Formulation 3** | 2.8 (±1.83) | 1012.84 (±916.19) | 5085.09 (±6086.84) |

**Table 5**

| ***24 h*** | **Tₘₐₓ (h)** | **Cₘₐₓ (ng/mL)** | **AUC _{0-inf}_obs (ng/mL*h)** |
|---|---|---|---|
| **Formulation 1** | 6 (±0) | 1287.97 (±512.24) | 14193.28 (±5480.60)** |
| **Formulation 2** | 3 (±1.55) | 365.07 (±91.08) | 3466.98 (±1456.11)** |
| **Formulation 3** | 2.8 (±1.83) | 1012.84 (±916.19) | 8861.71 (±12486.97) |

**Table 6**

| | **Formulation 1** | **Formulation 2** | **Formulation 3** |
|---|---|---|---|
| **Time after administration (h)** | **[CBD 2] (ng/mL) in plasma** | **[CBD 2 ] (ng/mL) in plasma** | **[CBD 2 ] (ng/mL) in plasma** |
| **0** | 0 | 0 | 0 |
| **0.25** | 1.81 (±3.82) | 69.74 (±36.75) | 197.94 (±112.75) |
| **0.5** | 11.6 (±5.18) | 114.42 (±78.48) | 274.91 (±151.91) |
| **1** | 46.64 (±26.40) | 148.14 (±83.29) | 402.44 (±148.01) |
| **2** | 93.06 (±33.08) | 284.48 (±35.86) | 234.19 (±112.85) |
| **3** | 93.03 (±30.52) | 307.45 (±98.37) | 798.92 (±696.78) |
| **6** | 1287.97 (±512.24) | 216.78 (±149.54) | 603.34 (±1044.10) |
| **12** | 38.87 (±10.96) | 7.49 (±8.81) | 20.48 (±32.99) |

| **Time after administration (h)** | **[7-OH-CBD 3] (ng/mL) in plasma** | **[7-OH-CBD 3 ] (ng/mL) in plasma** | **[7-OH-CBD 3 ] (ng/mL) in plasma** |
|---|---|---|---|
| **0** | 0 | 0 | 0 |
| **0.25** | 0 | 0 | 1.78 (±3.08) |
| **0.5** | 0 | 3.08 (±5.28) | 10.08 (±11.83) |
| **1** | 0.44 (±0.71) | 7.28 (±8.69) | 24.56 (±18.26) |
| **2** | 0.87 (±1.49) | 31.75 (±3.82) | 22.17 (±20.81) |
| **3** | 4.01 (±4.50) | 25.85 (±17.80) | 52.37 (±50.25) |
| **6** | 69.50 (±23.28) | 15.62 (±13.65) | 28.12 (±38.15) |
| **12** | --- | --- | --- |

| **Time after administration (h)** | **[7-OH-CBD 3/CBD 2] (ng/mL) in plasma** | **[7-OH-CBD 3/CBD 2] (ng/mL) in plasma** | **[7-OH-CBD 3/CBD 2] (ng/mL) in plasma** |
|---|---|---|---|
| **0** | 0 | 0 | 0 |
| **0.25** | 0 | 0 | 0.01 (±0.01) |
| **0.5** | 0 | 0.02 (±0.03) | 0.03 (±0.03) |
| **1** | 0.01 (±0.01) | 0.04 (±0.04) | 0.05 (±0.02) |
| **2** | 0.01 (±0.01) | 0.11 (±0.01) | 0.08 (±0.05) |
| **3** | 0.03 (±0.03) | 0.08 (±0.04) | 0.06 (±0.03) |
| **6** | 0.06 (±0.01) | 0.06 (±0.04) | 0.04 (±0.04) |
| **12** | --- | --- | --- |

## Claims

1. A composition in solid dispersion form comprising 2-[(1R,6R)-3-methyl-6-(prop-1-en-2-yl)-cyclohex-2-enyl]-5-pentylbenzen-1,3-diol (CBD 2) and at least two excipients, wherein at least one excipient ("excipient L") is a lipid excipient which is a sunflower or soy lecithin containing phosphatidylcholine in the amount of not less than 25% and wherein at least one excipient ("excipient I") is a hydrophilic excipient, wherein excipient L is present in an amount equal to or lower than 20% of the total weight of the composition.

2. The composition according to claim 1, wherein the hydrophilic excipient is present in an amount equal to or higher than 40% of the total weight of the composition.

3. The composition according to claims 1 and 2, wherein the lipid excipient is selected from the following: monoglycerides, diglycerides, triglycerides, fatty acids, lecithins and/or combinations thereof.

4. The composition according to claims 1-3, wherein the hydrophilic excipient is selected from cellulose, microcrystalline cellulose, methylcellulose, ethylcellulose, hydroxymethyl cellulose, hydroxyethyl cellulose, hydroxypropyl cellulose, hydroxypropylmethyl cellulose, carboxymethyl cellulose, carboxymethylethyl cellulose, starch, pectins, maltodextrins, cyclodextrins, sucrose, fructose, sorbitol, mannitol, maltitol, xylitol, dextrose, trehalose, inulin, carrageenan, alginate, gum arabic, guar gum and mixtures thereof.

5. A composition according to claims 1-4, further comprising a surfactant.

6. The composition according to claim 5, wherein the surfactant is selected from cetyl alcohols, glycerol monostearates, polyethylene glycol stearates, polysorbates, polyoxyethylene/polyoxypropylene copolymer, d-alpha tocopheryl polyethylene glycol 1000 succinate, polysorbates, PEG 40 hydrogenated castor oil and mixtures thereof.

7. A composition according to claims 1-6, wherein the weight ratio of CBD 2 to lipid excipient is higher than or equal to 0.5 and less than 10.

8. A composition according to claims 1-7, wherein the weight ratio of CBD 2 to hydrophilic excipient ranges between 0.1 and 10.

9. A composition according to claims 1-8, wherein the weight ratio of CBD 2 to surfactant ranges between 0.5 and 10.

10. Process for the preparation of the composition of claims 1-9 comprising the use of an organic solvent.

11. The process according to claim 10, comprising the following steps:
a) the lipid excipient is suspended in an organic solvent to give a suspension (suspension A);
b) suspension A is heated and kept under stirring until dissolution of the lipid excipient, to give a solution (solution B);
c) solution B is heated and kept under stirring, and CBD 2 is added to give a solution (solution C);
d) solution C is heated and kept under stirring, and the hydrophilic excipient is added to give a suspension (suspension D);
e) the solvent of suspension D is removed, to give a solid (solid E);
f) optional addition of a lubricant and/or glidant to solid E, to give a solid (solid F);
g) optional grinding of solid E or F to give a powder.

12. The process according to claims 10 and 11, wherein a further surfactant is added in step a).

13. Oral pharmaceutical or nutraceutical formulations containing the composition of any one of claims 1-9.

## Patentansprüche

1. Zusammensetzung in Form einer festen Dispersion, umfassend 2-[(1R,6R)-3-Methyl-6-(propen-2-yl)-cyclohex-2-enyl]-5-pentylbenzol-1,3-diol (CBD 2) und mindestens zwei Hilfsstoffe, wobei mindestens ein Hilfsstoff ("Hilfsstoff L") ein Lipidhilfsstoff ist, der ein Sonnenblumen- oder Sojalecithin ist, das Phosphatidylcholin in einer Menge von nicht weniger als 25 % enthält, und wobei mindestens ein Hilfsstoff ("Hilfsstoff I") ein hydrophiler Hilfsstoff ist, wobei Hilfsstoff L in einer Menge von 20 % oder weniger des Gesamtgewichts der Zusammensetzung vorliegt.

2. Zusammensetzung gemäß Anspruch 1, wobei der hydrophile Hilfsstoff in einer Menge von 40 % oder mehr des Gesamtgewichts der Zusammensetzung vorliegt.

3. Zusammensetzung gemäß den Ansprüchen 1 und 2, wobei der Lipid-Hilfsstoff aus den folgenden ausgewählt ist: Monoglyceride, Diglyceride, Triglyceride, Fettsäuren, Lecithine und/oder Kombinationen davon.

4. Zusammensetzung gemäß den Ansprüchen 1-3, wobei der hydrophile Hilfsstoff ausgewählt ist aus Cellulose, mikrokristalliner Cellulose, Methylcellulose, Ethylcellulose, Hydroxymethylcellulose, Hydroxyethylcellulose, Hydroxypropylcellulose, Hydroxypropylmethylcellulose, Carboxymethylcellulose, Carboxymethylethylcellulose, Stärke, Pektine, Maltodextrine, Cyclodextrine, Saccharose, Fructose, Sorbit, Mannit, Maltit, Xylit, Dextrose, Trehalose, Inulin, Carrageen, Alginat, Gummi arabicum, Guarkernmehl und Mischungen davon.

5. Zusammensetzung gemäß den Ansprüchen 1 bis 4, ferner umfassend ein Tensid.

6. Zusammensetzung gemäß Anspruch 5, wobei das Tensid ausgewählt ist aus Cetylalkoholen, Glycerinmonostearaten, Polyethylenglykolstearaten, Polysorb-Polyoxyethylen/Polyoxypropylen-Copolymeren, d-alpha-Tocopheryl-Polyethylenglykol-1000-succinat, Polysorbaten, PEG 40-hydriertem Rizinusöl und Mischungen davon.

7. Zusammensetzung gemäß den Ansprüchen 1 bis 6, wobei das Gewichtsverhältnis von CBD 2 zu Lipid-Trägerstoff größer oder gleich 0,5 und kleiner 10 ist.

8. Zusammensetzung gemäß den Ansprüchen 1 bis 7, wobei das Gewichtsverhältnis von CBD 2 zu hydrophilem Hilfsstoff zwischen 0,1 und 10 liegt.

9. Zusammensetzung gemäß den Ansprüchen 1 bis 8, wobei das Gewichtsverhältnis von CBD 2 zu Tensid zwischen 0,5 und 10 liegt.

10. Verfahren zur Herstellung der Zusammensetzung gemäß den Ansprüchen 1 bis 9, umfassend die Verwendung eines organischen Lösungsmittels.

11. Verfahren gemäß Anspruch 10, umfassend die folgenden Schritte:
a) der Lipid-Hilfsstoff wird in einem organischen Lösungsmittel suspendiert, um eine Suspension (Suspension A) zu erhalten;
b) Suspension A wird erhitzt und unter Rühren gehalten, bis sich der Lipid-Hilfsstoff aufgelöst hat, um eine Lösung (Lösung B) zu erhalten;
c) Lösung B wird erhitzt und unter Rühren gehalten, und CBD 2 wird zugegeben, um eine Lösung (Lösung C) zu erhalten;
d) Lösung C wird unter Rühren erhitzt, und der hydrophile Hilfsstoff wird zugegeben, um eine Suspension zu erhalten (Suspension D);
e) das Lösungsmittel der Suspension D wird entfernt, um einen Feststoff (Feststoff E) zu erhalten;
f) optionaler Zusatz eines Gleitmittels und/oder eines Trennmittels zu Feststoff E, um einen Feststoff (Feststoff F) zu erhalten;
g) optionales Mahlen des Feststoffs E oder F, um ein Pulver zu erhalten.

12. Verfahren gemäß den Ansprüchen 10 und 11, wobei in Schritt a) ein weiteres Tensid zugegeben wird.

13. Orale pharmazeutische oder nutrazeutische Formulierungen, die die Zusammensetzung gemäß einem der Ansprüche 1 bis 9 enthalten.

## Revendications

1. Composition sous la forme d'une dispersion solide comprenant du 2-[(1R,6R)-3-méthyl-6-(prop-1-én-2yl)-cyclohex-2-ényl]-5-pentylbenzène-1,3-diol (CBD 2) et au moins deux excipients ; dans laquelle au moins un excipient (« excipient L ») est un excipient lipidique à base de tournesol ou de lécithine de soja contenant de la phosphatidylcholine dans la quantité qui n'est pas inférieure à 25 % et dans laquelle au moins un excipient (« excipient I ») est un excipient hydrophile ; dans laquelle l'excipient L est présent en une quantité qui est égale ou inférieure à 20 % du poids total de la composition.

2. Composition selon la revendication 1, dans laquelle l'excipient hydrophile est présent en une quantité qui est égale ou supérieure à 40 % du poids total de la composition.

3. Composition selon les revendications 1 et 2, dans laquelle l'excipient lipidique est choisi parmi les suivants : des monoglycérides, des diglycérides, des triglycérides, des acides gras, des lécithines et/ou leurs combinaisons.

4. Composition selon les revendications 1 à 3, dans laquelle l'excipient hydrophile est choisi parmi la cellulose, la cellulose microcristalline, la méthylcellulose, l'éthylcellulose, l'hydroxyméthyl cellulose, l'hydroxyéthyl cellulose, l'hydroxypropyl cellulose, l'hydroxypropylméthyl cellulose, la carboxyméthyl cellulose, la carboxyméthyléthyl cellulose, l'amidon, les pectines, les maltodextrines, les cyclodextrines, le saccharose, le fructose, le sorbitol, le mannitol, le maltitol, le xylitol, le dextrose, le tréhalose, l'inuline, le carraghénane, l'alginate, la gomme arabique, la gomme de guar, ainsi que leurs mélanges.

5. Composition selon les revendications 1 à 4, qui comprend en outre un agent tensioactif.

6. Composition selon la revendication 5, dans laquelle l'agent tensioactif est choisi parmi des alcools cétyliques, des monostéarates de glycérol, des stéarates de polyéthylène glycol, des polysorbates, un copolymère de polyoxyéthylène/polyoxypropylène, du succinate de d-alpha tocophéryl polyéthylène glycol 1000, des polysorbates, de l'huile de ricin hydrogénée PEG 40, ainsi que leurs mélanges.

7. Composition selon les revendications 1 à 6, dans laquelle le rapport pondéral du CBD 2 à l'excipient lipidique est supérieur ou égal à 0,5 et inférieur à 10.

8. Composition selon les revendications 1 à 7, dans laquelle le rapport pondéral du CBD 2 à l'excipient hydrophile se situe dans la plage entre 0,1 et 10.

9. Composition selon les revendications 1 à 8, dans laquelle le rapport pondéral du CBD 2 à l'agent tensioactif se situe dans la plage entre 0,5 et 10.

10. Procédé pour la préparation de la composition selon les revendications 1 à 9, qui comprend l'utilisation d'un solvant organique.

11. Procédé selon la revendication 10, qui comprend les étapes suivantes au cours desquelles :
a) on met l'excipient lipidique en suspension dans un solvant organique afin d'obtenir une suspension (suspension A) ;
b) on chauffe la suspension A et on la maintient sous agitation jusqu'à dissolution de l'excipient lipidique, afin d'obtenir une solution (solution B) ;
c) on chauffe la solution B et on la maintient sous agitation, et on ajoute du CBD 2 afin d'obtenir une solution (solution C) ;
d) on chauffe la solution C et on la maintient sous agitation, et on ajoute l'excipient hydrophile afin d'obtenir une suspension (suspension D) ;
e) on élimine le solvant de la suspension D, afin d'obtenir un produit solide (produit solide E) ;
f) le cas échéant, on ajoute un lubrifiant et/ou agent de glissement au produit solide E, afin d'obtenir un produit solide (produit solide F) ;
g) le cas échéant, on broie le produit solide E ou F pour obtenir une poudre.

12. Procédé selon les revendications 10 et 11, dans lequel on ajoute un agent tensioactif supplémentaire à l'étape a).

13. Formulations nutraceutiques ou pharmaceutiques orales qui contiennent la composition selon l'une quelconque des revendications 1 à 9.
